(19) Europäisches Patentamt
European Patent Office
Office européen des brevets

(11) Veröffentlichungsnummer: **0 261 274**
**A1**

(12) # EUROPÄISCHE PATENTANMELDUNG

(21) Anmeldenummer: **86113288.4**

(51) Int. Cl.⁴: **C07C 149/20**

(22) Anmeldetag: **26.09.86**

(43) Veröffentlichungstag der Anmeldung:
**30.03.88 Patentblatt 88/13**

(84) Benannte Vertragsstaaten:
**AT BE CH DE FR GB IT LI LU NL SE**

(71) Anmelder: **C.F. Spiess & Sohn GmbH & Co. Chemische Fabrik**

**D-6719 Kleinkarlbach(DE)**

(72) Erfinder: **Spiess, Wolfram, Dr. Dipl.-Chem.**
**Heinrich-Becker-Strasse 16**
**D-6718 Grünstadt(DE)**
Erfinder: **Himmelreich, Rolf G.**
**Uhlandstrasse 9**
**D-6718 Grünstadt(DE)**

(74) Vertreter: **Eggert, Hans-Gunther, Dr.**
**Räderscheidtstrasse 1**
**D-5000 Köln 41(DE)**

(54) **Verfahren zur Herstellung von 3-Mercaptopropionsäure.**

(57) Durch Umsetzung von Natriumtrithiocarbonat in wässriger Lösung mit einem Acrylsäureester in einem weitgehend wasserunlöslichen organischen Lösungsmittel und Ansäuern mit einer nicht-oxydierenden Säure ist 3-Mercaptopropionsäure erhältlich. Das Verfahren bietet verfahrenstechnische und ökologische Vorteile.

EP 0 261 274 A1

# Verfahren zur Herstellung von 3-Mercaptopropionsäure

Die Erfindung betrifft ein Verfahren zur Herstellung von 3-Mercaptopropionsäure und/oder ihrer Ester, die industriell als Weichmacher, Stabilisatoren und Antioxydantien für Kunststoffe sowie zur Hochglanzmetallisierung in der Galvanotechnik eingesetzt werden.

Die Herstellung von 3-Mercaptopropionsäure erfolgt technisch analog zu der in der DE-PS 1 468 651 für Thioglykolsäure beschriebenen Methode. Dabei wird Natriumtrithiocarbonat mit dem Natriumsalz der 3-Chlorpropionsäure umgesetzt und beim Ansäuern 3-Mercaptopropionsäure erhalten, wie die folgende Gleichung zeigt

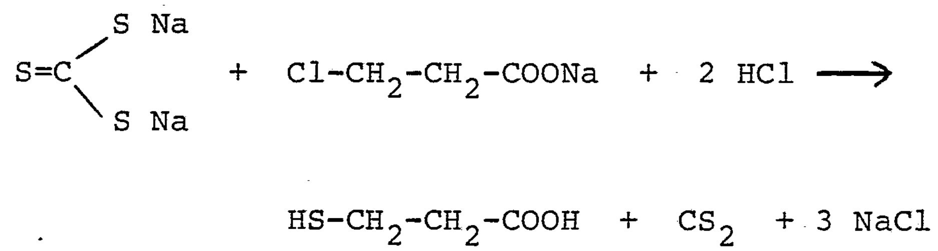

Bei diesem Verfahren entstehen pro Mol 3-Mercaptopropionsäure drei Mole Natriumchlorid. Das führt zu einer hohen Salzbelastung der Abwässer. Nachteilig bei diesem Verfahren ist ferner der hohe Preis des Ausgangsproduktes 3-Chlorpropionsäure.

Nach der US-PS 2 623 066 ist 3-Mercaptopropionsäure, ausgehend von Methylacrylat, zugänglich. Das Methylacrylat wird zunächst mit wässriger Natriumtetrasulfidlösung und anschließend mit Salzsäure umgesetzt.

Die erhaltene Reaktionslösung wird mit Aluminium und Natriumhydroxid behandelt. Unter verfahrensökonomischen Gesichtspunkten ist dieser Herstellungsweg als umständlich zu betrachten.

Der vorliegenden Erfindung liegt die Aufgabe zugrunde, ein Verfahren zur Herstellung von 3-Mercaptopropionsäure bereitzustellen, welches gegenüber den Verfahren nach dem Stand der Technik verfahrenstechnische Vorteile aufweist und die Salzbelastung des Abwassers verringert.

Es wurde nun überraschend ein Verfahren zur Herstellung von 3-Mercaptopropionsäure gefunden, welches die aufgeführten Nachteile der bekannten Verfahren vermeidet, indem man ein wasserlösliches Salz der Trithiokohlensäure in wässriger Lösung mit der Lösung einer etwa äuqimolaren Menge eines niederen Acrylsäureesters in einem weitgehend wasserunlöslichen organischen Lösungsmittel umsetzt und das Reaktionsgemisch ansäuert.

Ungesättigte Carbonsäuren, ihre Salze und Ester bilden mit aktiven Schwefelverbindungen wie $H_2S$, NaSH oder $Na_2S$ sowie mit den Sulfiden anderer Alkalimetalle leicht Thiodicarbonsäuren, weil die primär gebildete Mercaptocarbonsäure sofort mit noch vorhandener ungesättigter Carbonsäure reagiert. So entstehen aus Acrylsäure und Acrylsäureestern stets Thiodipropionsäure oder ihre Ester (Beilstein, 3. Ergänzungswerk, Bd. 3, Seite 552).

Bei der vorliegenden Erfindung wird die Bildung von Thiodipropionsäure und ihren Estern dadurch vermieden, daß

1. die Umsetzung in einem Zweiphasensystem erfolgt und
2. durch die Verwendung von Natriumtrithiocarbonat die primär gebildete Schwefelverbindung der Propionsäure bzw. ihres Esters nicht mit dem Acrylsäurealkylester weiter reagieren kann.

Die erfindungsgemäße Herstellung von 3-Mercaptopropionsäure verläuft nach folgenden Gleichungen:

(I)

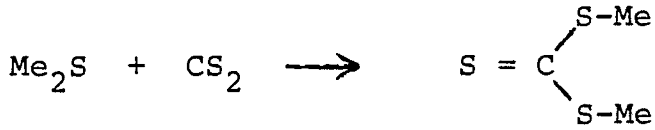

(II)

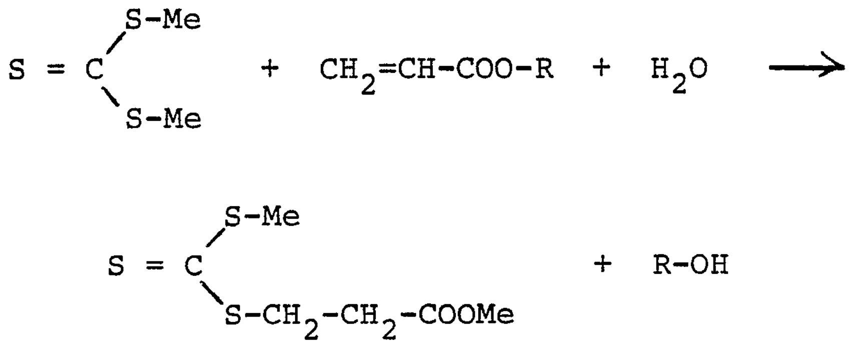

(III)

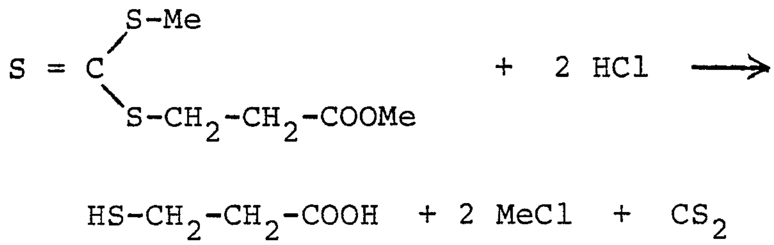

Hierin bedeutet Me ein Alkalimetall oder Ammonium, vorzugsweise Natrium, und R einen niederen Alkylrest mit 1 bis 4 Kohlenstoffatomen.

Wie aus den Gleichungen ersichtlich ist, wird der zur Bildung des Alkalitrithiocarbonats nach der Gleichung I eingesetzte Schwefelkohlenstoff gemäß Gleichung III bei der Zersetzung des Dinatrium-trithiocarbonyl-3-propionats oder des Natrium-trithiocarbonyl-3-propionsäureesters wieder frei. In der Praxis wird er vorzugsweise im Kreislauf wieder zur Bildung des Alkalitrithiocarbonats eingesetzt. Dabei auftretende geringe Verluste werden durch bedarfsweise Zugabe von frischem Schwefelkohlenstoff ausgeglichen.

Wie die Gleichungen ferner zeigen, werden bei der erfindungsgemäßen Herstellung von 3-Mercaptopropionsäure also pro Mol der Säure nur zwei Mole Natriumchlorid gebildet. Damit verringert sich die Salzbelastung des Abwassers um ein Drittel.

Zunächst wird nach Gleichung I in bekannter Weise ein wasserlösliches Alkalisulfid, vorzugsweise Natriumsulfid, mit Schwefelkohlenstoff zu dem entsprechenden Trithiocarbonat umgesetzt. Es hat sich als günstig erwiesen, hierbei den Schwefelkohlenstoff in einem Überschuss von 10 bis 40 % einzusetzen. Dies ist eine langsam ablaufende, schwach exotherme Reaktion. Zur Vervollständigung der Umsetzung wird daher nach dem Zutropfen des Schwefelkohlenstoffs mindestens noch eineinhalb Stunden weiter gerührt.

Zur Durchführung der Reaktion nach Gleichung II wird der Acrylsäurealkylester in einem inerten organischen Lösungsmittel gelöst und in dieser Lösung unter Rühren der wässrigen Lösung des Natriumtrithiocarbonats tropfenweise zugesetzt. Auch diese Reaktion verläuft langsam und schwach exotherm. Die Temperatur sollte während der Reaktion zwischen 0° bis 50°C, vorzugsweise 15 bis 35°C betragen. Bei 15°C liegt auch das Optimum für die Reaktion von Natriumtrithiocarbonat mit Acrylsäuremethylester. Anschließend wird noch mindestens 90 Minuten weiter gerührt. Man kann während des Zutropfens auch zusätzlich erwärmen, Temperaturen über 50°C verringern jedoch die Ausbeute. Außerdem nimmt bei höheren Temperaturen die Menge an Nebenprodukten, hauptsächlich Thiocarbonyl-bis(3-thiopropionsäure), zu.

Durch den Zusatz eines weitgehend wasserunlöslichen Lösungsmittels zum Acrylsäuremethylester wird der Übergang des Acrylsäureesters in die wässrige Phase und damit die Bildung von Thiodipropionsäure bzw. Thiodipropionsäurediester erschwert. Außerdem wirkt sich der Verdünnungseffekt günstig auf den Verlauf der Reaktion aus. Am besten als Lösungsmittel geeignet sind Ether, wie Diethyl-oder Diisopropylether, alkylierte aromatische Kohlenwasserstoffe, wie Toluol oder Xylol, oder geradkettige aliphatische $C_5$-$C_{12}$-Kohlenwasserstoffe.

Grundsätzlich ist es auch möglich, das Lösungsmittel direkt zur wässrigen Phase zu geben und den unverdünnten Acrylsäureester zuzutropfen. Da der Verdünnungseffekt zumindest an der Eintropfstelle teilweise aufgehoben wird, wird durch diese Reaktionsführung allerdings die Ausbeute etwas geringer.

Die Ausbeute ist außerdem von der Konzentration des Natriumtrithiocarbonats abhängig, die nicht größer als 1 Mol Natriumtrithiocarbonat in 500 ml Wasser sein sollte.

Die Ausbeute ist schließlich auch von der Konzentration des Acrylsäureesters im Lösungsmittel abhängig. Für Ether liegt das Optimum bei 150 bis 300 ml für 1 Mol Acrylsäuremethylester.

Bei der Anlagerung des Natriumtrithiocarbonats an die Doppelbindung des Acrylsäureesters nach Gleichung II wird zugleich die Estergruppe ganz oder teilweise verseift. Dementsprechend werden wechselnde Mengen an 3-Mercaptopropionsäureestern als Nebenprodukt erhalten.

Je größer die Alkylgruppe des Esters desto geringer ist die Verseifung, aber umso größer auch die Bildung von Nebenprodukten. Die besten Ausbeuten an freier Säure werden daher mit dem Methylester erhalten, der zugleich am preisgünstigten ist und infolge seines niedrigen Molekulargewichts die einzusetzenden Mengen und Volumina minimiert. Vorteilhaft können auch andere niedrige Alkylester der Acrylsäure, z.B. der Ethyl-, Isopropyl-und Butylester eingesetzt werden. Dabei ergibt der Ethylester fast ebenso gute Gesamtausbeuten wie der Methylester, aber mit einem hohen Anteil des 3-Mercaptopropionsäureethylesters.

Im letzten Verfahrensschritt III wird das Reaktionsgemisch mit einer unter den Reaktionsbedingungen nicht oxidierenden Säure, die nur stärker sein muß als die 3-Mercaptopropionsäure bis auf einen pH-Wert von vorzugsweise 1 angesäuert. Die Wahl der Säure hängt vorwiegend von den Möglichkeiten der Entsorgung oder Verwertung des nach Gleichung III anfallenden Salzes ab. Vorzugsweise wird wie bei dem bekannten technischen Verfahren zur Herstellung von 3-Mercaptopropionsäure Salzsäure für die Zersetzung des Dinatrium-trithiocarbonyl-3-propionats verwendet.

Nach Abtrennen der organischen Phase wird die wässrige Phase mit Ether extrahiert, die organischen Phasen werden vereinigt und die Lösungsmittel abdestilliert. Der Rückstand wird im Vakuum fraktioniert. Es werden 80 bis 90% 3-Mercaptopropionsäure und bis zu 15 % 3-Mercaptopropionsäurealkylester erhalten, die wie das Hauptprodukt nach Destillation technisch verwendbar sind.

Der beim Ansäuern freigesetzte Schwefelkohlenstoff wird im allgemeinen mit dem Lösungsmittel azeotrop bei Temperaturen um 40°C abdestilliert. Zur Wiederverwendung des Schwefelkohlenstoffs wird die Menge des $CS_2$ im Gemisch bestimmt und mit der entsprechenden Menge einer wässrigen Alkalisulfidlösung zu Alkalitrithiocarbonat umgesetzt. Anschließend wird dann das reine Lösungsmittel abgetrennt und darin wieder frischer Acrylsäuremethylester für die weitere Herstellung von 3-Mercaptopropionsäure gelöst.

Die Erfindung wird anhand der folgenden Beispiele verdeutlicht:

## Beispiel 1

220 g 60%iges Natriumsulfid werden mit 500 ml Wasser versetzt. Unter mechanischem Rührer werden im Verlauf von 40 min am Rückflusskühler 140 ml Schwefelkohlenstoff zugetropft und anschließend 3 h gerührt. Dann wird mit fließendem Wasser gekühlt und die Lösung von 160 g Acrylsäuremethylester in 300 ml Ether mit solcher Geschwindigkeit zugetropft, daß die Temperatur nicht über + 15°C ansteigt. Anschließend wird noch 4 h ohne Kühlung weiter gerührt. Die Apparatur wird schließlich mit einer mit Natron lauge beschickten Absorptionsanlage verbunden und unter erneutem Kühlen mit fließendem Wasser werden 400 ml konzentrierte Salzsäure zugetropft. Die Mischung sollte dann einen pH-Wert von ca. 1 haben. Die organische Phase wird in einem Scheidetrichter abgetrennt, die wässrige Phase wird in einer Extraktionsapparatur mit Ether extrahiert. Aus den vereinigten organischen Phasen wird der Ether im azeotropen Gemisch mit Schwefelkohlenstoff abdestilliert. Der Rückstand wird im Vakuum fraktioniert. Bei $Kp_{14}$ 87 bis 88°C destillieren 17,7 g 3-Mercaptopropionsäuremethylester. Der Ester hat einen Gehalt von 100,6 %, er enthält Spuren von 3-Mercaptopropionsäure. Die Ausbeute beträgt 8,7 % der Theorie. Als Hauptfraktion wird 3-Mercaptopropionsäure erhalten. Bei $Kp_{0,18}$ 76 bis 82°C destillieren 148,2 g 3-Mercaptopropionsäure über, das sind 82,6 % der Theorie. Die Substanz hat einen Gehalt von 99,8 %. Es verbleiben 13,7 g eines gelben, nicht destillierbaren Rückstandes von Thiocarbonyl-bis-(3-thiopropionsäure), der beim Abkühlen auskristallisiert. Die Gesamtausbeute an freier Säure und ihrem Methylester, bezogen auf das eingesetzte Natriumtrithiocarbonat, beträgt also 91,3 %.

Beispiel 2

130 g 60%iges Natriumsulfid werden zu 265 ml Wasser gegeben. Unter mechanischem Rühren am Rückfluß tropft man dazu im Verlauf von 20 min 70 ml Schwefelkohlenstoff. Die Mischung wird 2 h weiter gerührt. Dann tropft man im Verlauf von 1 h die Lösung von 100 ml Acrylsäure Ethylester in 150 ml Ether dazu. Die Mischung wird 3 h weiter gerührt. Dann wird mit fliessendem Wasser gekühlt und mit einer Absorptionseinheit, die mit Natronlauge beschickt ist, verbunden. Es werden 220 ml konzentrierte Salzsäure zugetropft, die Lösung soll einen pH-Wert von ca. 1 haben. Die organische Phase wird abgetrennt. Die wässrige Phase wird mit Ether extrahiert. Aus den vereinigten organischen Phasen wird das Ether/Schwefelkohlenstoff-Gemisch abdestilliert. Der Rückstand wird fraktioniert. Es werden 11,0 g eines Destillats bei Kp $_{12}$ 55°C erhalten, bei dem es sich um ein Gemisch von 64,3 % Acrylsäureethylester, 27,9 % 3 Mercaptopropionsäureethylester und 7,8 % 3-Mercaptopropionsäure handelt. Bei Kp $_{1,5}$ 90°C destillieren 81,8 g 3-Mercaptopropionsäure mit einem Gehalt von 99,1 %, entsprechend 77,2 % der Theorie. Die Gesamtausbeute an freier Säure und ihrem Ethylester, bezogen auf Natriumtrithiocarbonat beträgt also 79,5 %.

Beispiel 3

Das Beispiel wird unter den in Beispiel 2 angegebenen Bedingungen durchgeführt, jedoch werden anstelle von 100 g Acrylsäureethylester 86 g Acrylsäuremethylester und anstelle von 150 ml Ether 150 ml Diisopropylether eingesetzt. Bei diesem Versuch wird kein 3-Mercaptopropionsäuremethylester erhalten. Es werden 74,8 g 3-Mercaptopropionsäure bei Kp $_{0,3}$ 84°C erhalten, die einen Gehalt von 99,7 % aufweist, entsprechend 70,6 % der Theorie.

Beispiel 4

Das Beispiel wird unter den gleichen Bedingungen wie in Beispiel 3 durchgeführt, jedoch werden anstelle von 150 ml Ether 150 ml Hexan eingesetzt. Bei Kp $_{15}$ 90°C werden 13,5 g 3-Mercaptopropionsäuremethylester erhalten, das sind 11,25 % der Theorie. Der Ester hat einen Gehalt von 97,6 %. Der Rest ist 3-Mercaptopropionsäure.

Es werden außerdem bei Kp $_{0,05}$ 72°C 55,3 g 3-Mercaptopropionsäure mit einem Gehalt von 98,7 % erhalten. Die Ausbeute an 3-Mercaptopropionsäure beträgt 52,2 % der Theorie. Die Gesamtausbeute an freier Säure und ihrem Methylester, bezogen auf eingesetztes Natriumtrithiocarbonat beträgt 63,75 %.

**Ansprüche**

1. Verfahren zur Herstellung von 3-Mercaptopropionsäure und ggf. ihren Estern, dadurch gekennzeichnet, daß man ein wasserlösliches Salz der Trithiokohlensäure in wässriger Lösung mit der Lösung einer etwa äquimolaren Menge eines niederen Acrylsäureesters in einem weitgehend wasserunlöslichen organischen Lösungsmittel umsetzt und das Reaktionsgemisch ansäuert.

2. Verfahren nach Anspruch 1, dadurch gekennzeichnet, daß das wasserlösliche Salz der Trithiokohlensäure das Natriumtrithiocarbonat ist.

3. Verfahren nach Anspruch 1 oder 2, dadurch gekennzeichnet, daß der verwendete niedere Acrylsäureester der Methyl-, Ethyl-, i-Propyl-oder Butylester ist.

4. Verfahren nach einem der Ansprüche 1 bis 3, dadurch gekennzeichnet, daß als weitgehend wasserunlösliches organisches Lösungsmittel ein Ether, ein alkylierter aromatischer Kohlenwasserstoff, wie Toluol oder Xylol, oder ein gesättigter aliphatischer oder cycloaliphatischer $C_5$-$C_{12}$-Kohlenwasserstoff verwendet wird.

5. Verfahren nach einem der Ansprüche 1 bis 4, dadurch gekennzeichnet, daß die Umsetzung zwischen dem Trithiocarbonat und dem Acrylsäureester bei Temperaturen von 0 bis 50°C, vorzugsweise zwischen 15 und 35°C, durchgeführt wird.

6. Verfahren nach einem der Ansprüche 1 bis 5, dadurch gekennzeichnet, daß das Ansäuern mit einer nicht-oxidierenden Säure, insbesondere Salzsäure, erfolgt.

7. Verfahren nach einem der Ansprüche 1 bis 6, dadurch gekennzeichnet, daß der beim Ansäuern des Trithiokohlensäure-Monoesters freiwerdende Schwefelkohlenstoff im Kreislauf wieder zur Bildung des wasserlöslichen Salzes der Trithiokohlensäure eingesetzt wird.

8. Verfahren nach einem der Ansprüche 1 bis 7, dadurch gekennzeichnet, daß der eingesetzte Schwefelkohlenstoff im Kreislauf gefahren wird.

Europäisches Patentamt

# EUROPÄISCHER RECHERCHENBERICHT

Nummer der Anmeldung

EP 86 11 3288

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 097 791 (DEGUSSA) * Anspruch 1, Seite 4, Absätze 1, 2 * --- | 1-4 | C 07 C 149/20 |
| A | DE-B-2 244 234 (SANKYO) ----- | 1 | |

| RECHERCHIERTE SACHGEBIETE (Int. Cl.4) |
|---|
| C 07 C 149/20 |

Der vorliegende Recherchenbericht wurde für alle Patentansprüche erstellt.

| Recherchenort | Abschlußdatum der Recherche | Prüfer |
|---|---|---|
| BERLIN | 11-05-1987 | KAPTEYN H G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82